# FASCICULE DE BREVET EUROPEEN

(11) **EP 3 565 485 B1**
(45) Date de publication et mention de la délivrance du brevet: **02.03.2022**
(21) Numéro de dépôt: 18701514.4
(22) Date de dépôt: 03.01.2018
(51) Int. Cl.: A61B 17/122, A61B 17/128

(54) **AGRAFE CHIRURGICALE PRESENTANT DEUX BRANCHES MOBILES RELIEES PAR UNE ZONE DE LIAISON TRANSVERSALE**
CHIRURGISCHE KLAMMER MIT ZWEI BEWEGLICHEN ARMEN, DIE DURCH EINEN QUERVERBINDUNGSBEREICH VERBUNDEN SIND
SURGICAL STAPLE HAVING TWO MOVABLE BRANCHES CONNECTED BY A TRANSVERSE CONNECTING ZONE

(30) Priorité: 03.01.2017 FR 1750041
(43) Date de publication de la demande: 13.11.2019
(73) Titulaire: Institut Hospitalo-Universitaire de Chirurgie Mini -Invasive Guidee Par l'Image, 67091 Strasbourg (FR)
(72) Inventeur: ALZAGA, Amilcar, Del.Benito Juarez, Mexico D.F. 03710 (MX); HALVAX, Peter, 7635 Pecs (HU); SWANSTRÖM, Lee L, Portland, Oregon 97210 (US); HERNANDEZ, Juan, 67091 Strasbourg (FR)
(74) Mandataire: IP Trust
(86) Numéro de dépôt international: PCT/FR2018/050014
(87) Numéro de publication internationale: WO 2018/127659

(56) Documents cités:
- WO-A1-02/19920
- WO-A1-2009/135009
- WO-A1-2016/128693
- WO-A2-2016/128691
- US-A1- 2009 093 826
- US-A1- 2011 144 691

## Description

### Domaine de l'invention

La présente invention concerne une agrafe pour la suture et la ligature internes ainsi que la pose d'implants fixés sur un tissu par des agrafes durant une chirurgie minimalement invasive.

Ces agrafes constituées d'un matériau biocompatible métallique (le plus souvent en inox) sont utilisées en chirurgie endoscopique ou laparoscopique pour maintenir les deux lèvres tissulaires en contact ou pour la fixation d'une prothèse telle qu'un stent.

De telles agrafes présentent une mâchoire déformable définie par une séparation entre les deux segments.

Elles doivent présenter un encombrement en position déployée compatible avec le passage dans le tube endoscopique et sont manipulées avec un applicateur placé au bout du tube endoscopique. Contrairement aux agrafes destinées à une application cutanée, osseuse ou oculaire, les agrafes endoscopiques doivent permettre un positionnement et une fermeture dans un espace très réduit avec une faible possibilité de mouvement.

### Etat de la technique

On connaît dans l'état de la technique différentes solutions d'agrafes destinées à une pose endoscopique.

La demande de brevet américain US2011/0144691 propose une agrafe présentant une forme générale en « U » avec une base prolongée par deux jambes en forme de « L ». Les jambes sont pliées pour former un angle d'environ 90°. Elles présentent une portion pointue venant perforer les tissus. Elle présente aussi des structures de compression placées au-dessus de la portion de perforation limitant la profondeur de pénétration de la portion de perforation.

La demande internationale WO2016128691 de la demanderesse décrit une version antérieure d'une agrafe chirurgicale présentant deux branches mobiles reliées par une zone de liaison transversale. Chacune desdites branches présente une zone de pincement et une extrémité d'accrochage. La zone de liaison transversale est déformable pour permettre le rapprochement desdites branches mobiles par un pliage autour de l'axe de ladite zone de liaison transversale. Ce document décrit notamment une variante de réalisation d'une agrafe présentant des branches portant plusieurs crochets découpés dans la largeur du bras et recourbés, chacun des crochets présentant une surface de pincement.

La demande internationale WO2016128693 de la demanderesse décrit un système de chirurgie endoscopique formé d'une part par une pluralité d'agrafes et d'autre part par un applicateur endoscopique comportant un chargeur d'agrafes disposées transversalement caractérisé en ce que lesdites agrafes présentent une zone centrale et deux branches latérales et en ce que l'applicateur comporte un organe mobile présentant un moyen de retenue dans un plan transversal d'une agrafe à appliquer et pour assurer sa déformation par pliage par rapport à l'axe de symétrie de ladite agrafe.

D'autres exemples d'agrafes connues dans l'art antérieur sont décrits dans la demande de brevet américaine US2009/093826 ou dans les demandes de brevet internationales WO2002/19920 ou WO2009/135009.

### Inconvénients de l'art antérieur

Le but de l'invention est d'améliorer les agrafes chirurgicales de façon à assurer un maintien satisfaisant des tissus pincés sans écraser les tissus si l'effort appliqué est trop important, ni au contraire permettre un glissement si l'effort est insuffisant ou si le tissu évolue, et bien sur en évitant de léser les lèvres lors du déplacement de la zone de pincement.

La solution apportée par l'invention concerne une configuration particulière de ces dents, telle que revendiquée.

Pour des applications endoscopiques, l'agrafe doit présenter une très petite dimension, avec des structures d'une épaisseur maximale de moins d'un millimètre et d'une longueur de quelques millimètres.

Par ailleurs, les agrafes de l'art antérieur impliquent une perforation des lèvres tissulaires, provoquant ainsi une amorce de déchirure. Toutes les agrafes proposées dans l'art antérieur présentent des pointes pénétrant profondément dans les tissus pour en assurer le maintien, ce qui provoque des lésions du matériel cellulaire et des sources d'infection.

Enfin, la pression exercée sur les tissus par les agrafes de l'art antérieur pendant et après la fermeture est mal maitrisée et peut provoquer des dégradations voire une nécrose locale.

Il est donc très difficile de produire en séries industrielles des agrafes présentant une géométrie complexe.

Par ailleurs, il est difficile d'obtenir un maintien satisfaisant des tissus pincés avec une dent allongée, qui tend à écraser les tissus si l'effort appliqué est trop important, ou au contraire permettre un glissement si l'effort est insuffisant ou si le tissu évolue.

La solution proposée dans la variante « multicrochets » de la demande WO2016128691 n'est pas non plus totalement satisfaisante, car tous les efforts exercés sur le tissu sont produit par l'unique dent de pincement prévue sur chaque branche, et car la largeur des branches ne permet généralement pas de découper plus d'un crochet, sauf à élargir la branche, ce qui pose des problèmes d'encombrement.

### Solution apportée par l'invention

Afin de remédier à ces inconvénients, l'invention concerne selon son acception la plus générale une agrafe chirurgicale conforme à la revendication 1, et optionnellement à une ou plusieurs revendications dépendantes.

L'invention concerne aussi un système constitué par une agrafe chirurgicale conforme à la revendication 1 et un applicateur adapté pour être inséré au travers du canal opérateur d'un endoscope flexible, et comportant un crochet apte à coopérer avec la zone de pliage médiane, l'applicateur étant pourvu à son extrémité libre d'un manchon présentant une surface annulaire apte à former une butée contre laquelle viennent s'appuyer les épaulements formés par les surfaces des deuxièmes segments arqués, pour provoquer le rapprochement des deux bras par un pivotement autour du centre du segment arqué, symétriquement par rapport au plan médian lorsqu'on exerce une traction en direction opposée au manchon.

On entend par plan médian le plan qui passe par le centre du segment arqué réunissant les deux bras et par le milieu de la droite reliant les deux extrémités pointues. Généralement, ce plan médian constitue un plan de symétrie de l'agrafe, mais selon certaines variantes, les extrémités pointues ne sont pas symétriques par rapport à ce plan.

Selon une variante particulière, l'intérieur du tube est crénelé afin de permettre la transmission de la rotation à l'agrafe en faisant entrer la gaine externe en rotation.

### Description détaillée d'un exemple non limitatif de

### réalisation

La présente invention sera mieux comprise à la lecture de la description qui suit, se référant aux dessins annexés où :
- les figures 1 et 2 représentent une vue d'un premier exemple de réalisation ne faisant pas partie de l'invention respectivement de face et de 3/4 face, en position ouverte
- la figure 3 représente une vue de l'agrafe en position déployée partiellement engagée dans l'applicateur, avant la pose de l'agrafe
- la figure 4 représente une vue de l'agrafe et de l'applicateur après déformation de l'agrafe
- la figure 5 représente une vue d'une variante de réalisation ne faisant pas partie de l'invention
- la figure 6 représente une vue d'une autre variante de réalisation suivant l'invention
- les figures 7 et 8 représentent une vue et une vue de détail d'une variante de réalisation suivant l'invention
- la figure 9 représente une vue d'une autre variante de réalisation ne faisant pas partie de l'invention
- les figures 10 à 14 représentent des vues d'autres variantes de réalisation suivant l'invention.

### Description détaillée de l'agrafe

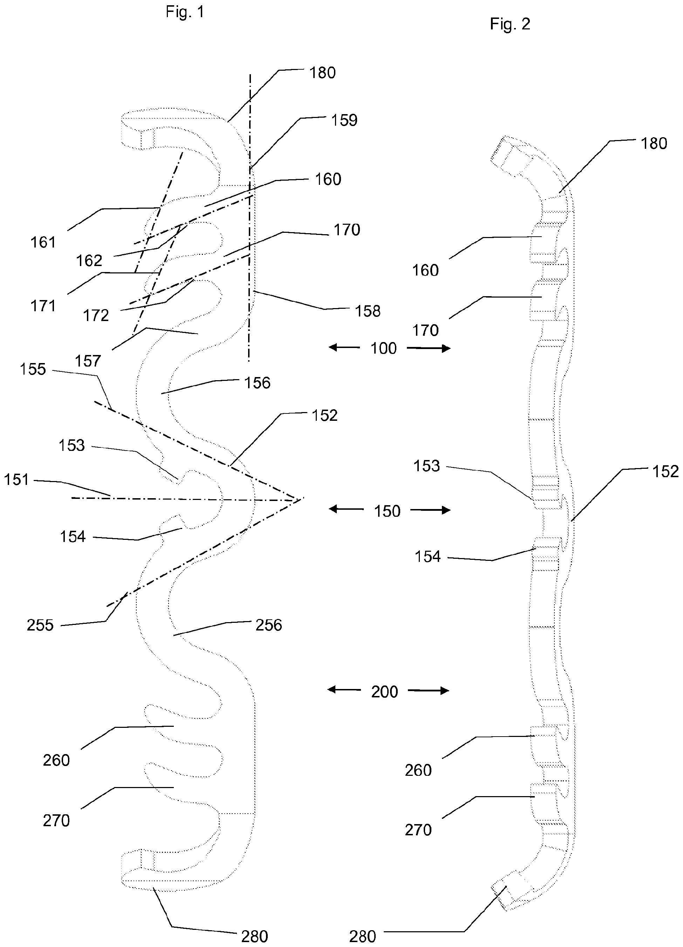

Les figures 1 et 2 représentent une vue schématique d'un premier exemple de réalisation respectivement de face et de 3/4 face, en position ouverte.

L'agrafe est représentée après découpe et repliement des extrémités, telle qu'elle se présente avant l'utilisation.

Elle comprend deux bras (100), (200) reliés par une zone médiane (150) de pliage.

L'agrafe est symétrique par rapport à un plan médian (151) perpendiculaire à la tôle dans laquelle est découpée l'agrafe, et passant au milieu de la zone médiane (150) de pliage.

La zone de pliage (150) présente un segment arqué (152) de forme semi-circulaire, s'étendant sur environ 90°. Ce segment arqué (152) présente à ses extrémités deux butées (153, 154) s'étendant perpendiculairement aux tangentes respectivement (155, 255). Ces tangentes (155, 255) correspondent à la tangente du segment arqué (152), au niveau des extrémités correspondant également à un point d'inflexion où le rayon de courbure change de sens.

Dans la suite, la description portera sur l'un des bras (100) étant entendu que l'autre bras présente, de manière symétrique, les mêmes caractéristiques.

Le segment arqué (152) se prolonge par un deuxième segment arqué (156) s'étendant sur environ 90°, avec une courbure inversée par rapport à la courbure du segment arqué (152) médian.

Ce deuxième segment arqué (156) se prolonge par un troisième segment arqué (157) s'étendant sur 45° environ, avec une courbure à nouveau inversée.

Ce troisième segment arqué (157) se prolonge par un segment sensiblement rectiligne (158) dont l'axe longitudinal (159) est perpendiculaire au plan de symétrie médian (151).

Ce segment rectiligne (158) présente deux dents (160, 170) s'étendant dans une direction sensiblement perpendiculaire par rapport à l'axe longitudinal (159).

Ces dents (160, 170) présentent des zones arquées (161, 171) dirigées du coté distal (opposé à la zone de pliage médiane (152)) avec une tangente formant un angle inférieur à 20° par rapport à un axe longitudinal parallèle à l'axe (159).

Du coté proximal, les dents (160, 170) présentent des zones arquées (162, 172) dont la tangente forme un angle compris entre 60° et 120° par rapport à un axe longitudinal parallèle à l'axe (159).

### Procédé de fabrication

L'agrafe selon l'invention est réalisée par une découpe d'une tôle métallique plane, par exemple par une découpe à jet d'eau. La tôle métallique présente une épaisseur constante d'environ un millimètre, nominalement entre 0,8 et 1,5 millimètres. Elle est réalisée en un matériau biocompatible, déformable sans élasticité, par exemple de l'acier inoxydable ou du titane.

A partir d'une découpe contenue dans un plan, l'agrafe peut présenter différents profils de courbures.

Ces courbures peuvent être réguliers, en fer à cheval par exemple ou présenter un profil avec des rayons de courbure variables, par exemple présentant des ruptures de courbe plus franches.

Ces différentes courbures générales vont être avantageuses selon le mode d'utilisation. Dans le cas d'une utilisation avec un applicateur adapté pour être inséré au travers du canal opérateur d'un endoscope flexible, la configuration minimisant l'enveloppe extérieure de l'agrafe sera favorisée. Dans le cas d'utilisation avec d'autres applicateurs, il peut être intéressant que l'agrafe se conforme à la forme de l'applicateur.

Après découpe de l'agrafe, le procédé de fabrication peut comporter des étapes de torsions de certaines zones pour les orienter dans des plans différents du plan principal.

### Coopération entre l'agrafe et l'applicateur

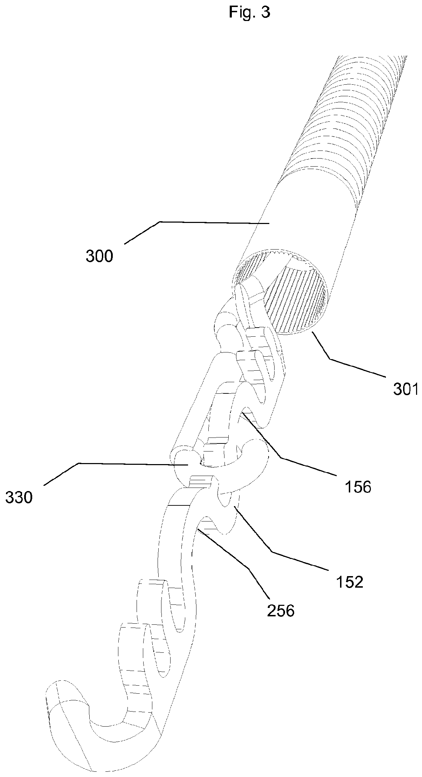

L'agrafe peut être introduite dans un manchon (300) tubulaire comme représenté en figure 3.

Elle est introduite dans l'applicateur (300) avec les deux bras écartés à environ 180°, pour permettre d'enfiler l'agrafe selon l'axe longitudinale de l'applicateur.

L'agrafe est maintenue par un crochet (330) qui vient s'engager dans la boucle définie par le segment arqué (152). Cet assemblage permet l'insertion et la rétraction de l'agrafe en position ouverte, dans le manchon (300), par un déplacement longitudinal.

Lorsque l'agrafe est totalement sortie du manchon (300), elle peut basculer dans une direction perpendiculaire, par un pivotement autour du crochet (330) engagé dans le segment (152). Les bras sont alors déployés, toujours à environ 180°, mais selon un axe perpendiculaire à l'axe de l'applicateur.

En exerçant ensuite une traction sur le crochet en direction du manchon (300), l'agrafe entre en contact avec la surface annulaire frontale (301), par l'intermédiaire des surfaces des segments arqués (156, 256). En exerçant un effort longitudinal, on provoque un repliement de l'agrafe par rapport à son plan médian (151).

La boucle sur laquelle est montée l'agrafe a une forme complexe contournant l'agrafe. L'intérieur du tube peut être crénelé afin de permettre la transmission de la rotation à l'agrafe en faisant entrer la gaine externe en rotation.

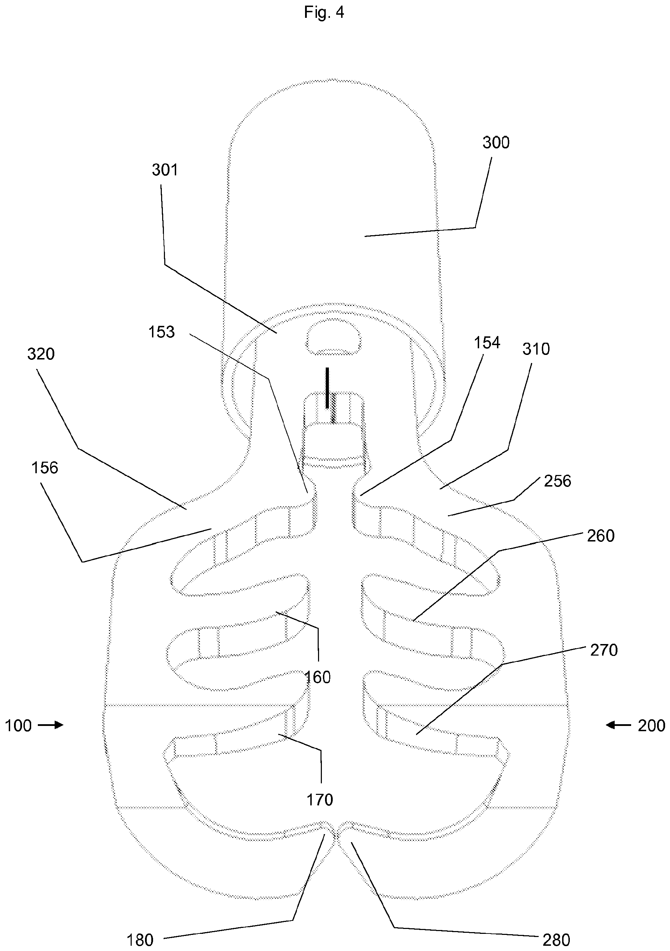

Lorsque l'agrafe est repliée par un basculement des bras (100, 200) par rapport au centre de la zone médiane (150), les dents (160, 170) de l'un des bras se rapproche des dents de l'autre bras pour former des zones de pincement entre les zones arquées (161, 171) comme représenté en figure 4.

Le bras (100) se termine par un crochet (180) formant une griffe d'accrochage. Ce crochet (180) est tordu par rapport au plan de la tôle, et présente un plan médian formant un angle de 30° environ par rapport au plan médian (151) des bras (100, 200).

Le deuxième bras (200) est symétrique au premier plan (100) par rapport au plan médian (151).

Lorsque l'agrafe est positionnée dans l'applicateur, le déplacement relatif du manchon (300) provoque le repliement de l'agrafe qui se déforme alors comme représenté en figure 4.

La surface annulaire frontale (301) du manchon (300) vient s'appuyer contre les épaulements (310, 320) formés par les surfaces des deuxièmes segments arqués (156, 256), ce qui provoque le rapprochement des deux bras (100, 200) par un pivotement autour du centre du segment arqué (152), symétriquement par rapport au plan médian (151).

Le rayon de courbure du segment médian (152) est légèrement inférieur au rayon intérieur du manchon (300).

Pendant ce mouvement de rapprochement des bras (100, 200), les deux crochets (180, 280) se déplacent sur une trajectoire arquée affleurant la surface des tissus à rapprocher et accrochent sans les perforer les deux surfaces pour les entraîner dans un déplacement tendant à les rapprocher et à former deux lèvres qui viennent se placer entre les dents (160, 170 ; 260, 270). Ces dents (160, 170 ; 260, 270) laissent un espace de 0,5 à 2 millimètres entre elles, lorsque les deux griffes (180, 280) viennent en contact, ce qui assure un maintien des deux lèvres des tissus à rapprocher par pincement, sans léser les tissus.

Cette solution permet d'avoir une agrafe dont l'enveloppe en position fermée est supérieure à l'enveloppe de l'applicateur, permettant ainsi de laisser un espace libre entre les jambes de l'agrafe jusqu'à la base de l'agrafe. Dans les solutions avec des jambes flexibles, comme les clips hémostatiques, l'espace intérieur s'affine progressivement, la géométrie ne contribuant pas à la compression mais ajoutant à la longueur générale de l'instrument (ce qui est une faiblesse car ceci gêne le praticien pour la suite de l'intervention après l'application).

Chaque bras (100, 200) peut comprendre plusieurs extrémités de pincement, dans l'une des réalisations favorisées pour une utilisation en endoscopie flexible, chaque jambe comprend deux extrémités de pincement. Les extrémités de pincement sont recourbées par rapport aux jambes et présente une surface non traumatique, cette surface faisant face à la surface équivalente placée sur l'autre jambe une fois l'agrafe refermée. Dans une utilisation dédiée à une compression de la muqueuse, un espace libre est décrit à l'intérieur de l'agrafe.

La présence d'une deuxième extrémité de pincement permet d'accroître la compression de la muqueuse et améliore la tenue de l'agrafe après application. Comme décrit au paragraphe précédent cette extrémité pourrait avoir d'autres géométries à partir du moment où deux surfaces atraumatiques se font face une fois l'agrafe fermée. Dans le cas d'une utilisation pour compression de la muqueuse, une géométrie décrivant un espace libre enceint entre les jambes de l'agrafe sera favorisé. Cette variante est particulièrement adaptée pour l'hémostase et la fermeture sur muqueuse.

### variante de réalisation

Cette variante diffère de l'exemple précédent par le nombre de dents.

Elle présente une zone médiane arquée (152) avec deux butées (153, 154) s'étendant perpendiculairement.

De part et d'autre de cette zone médiane arquée (152), l'agrafe présente des segments arqués (156, 256) recourbés en sens opposé. Ces segments arqués (156, 256) sont prolongés par des segments rectilignes respectivement (158, 258) s'étendant, avant pliage, selon un axe perpendiculaire au plan médian (151).

Ces segments rectilignes respectivement (158, 258) sont pourvus des dents respectivement (160, 165, 166, 170 ; 260 ; 265 ; 266 ; 270).

Les segments rectilignes respectivement (158, 258) sont prolongés par des griffes respectivement (180, 280).

De façon générale, l'invention ne se limite pas aux exemples décrits à titre non limitatifs, mais à différents profils de courbures obtenues à partir d'une découpe contenue dans un plan. Ces courbures peuvent être régulières, en fer à cheval par exemple, ou présenter un profil avec des rayons de courbure variables, par exemple présentant des ruptures de courbe plus franches.

Ces différentes courbures générales vont être avantageuses selon le mode d'utilisation. Dans le cas d'une utilisation avec un applicateur adapté pour être inséré au travers du canal opérateur d'un endoscope flexible, la configuration minimisant l'enveloppe extérieure de l'agrafe sera favorisée. Dans le cas d'utilisation avec d'autres applicateurs, il peut être intéressant que l'agrafe se conforme à la forme de l'applicateur.

Les extrémités de pincement et d'accrochage peuvent être pointues, biseautées mais aussi non pointues ou arrondies. Ces différentes configurations correspondent à différents types de tissus et à différents effets. Dans le cas d'une application sur de la muqueuse, des extrémités biseautés non pointues seront suffisantes pour réaliser l'ancrage, dans le cas de l'épiderme des extrémités pointues seront nécessaires.

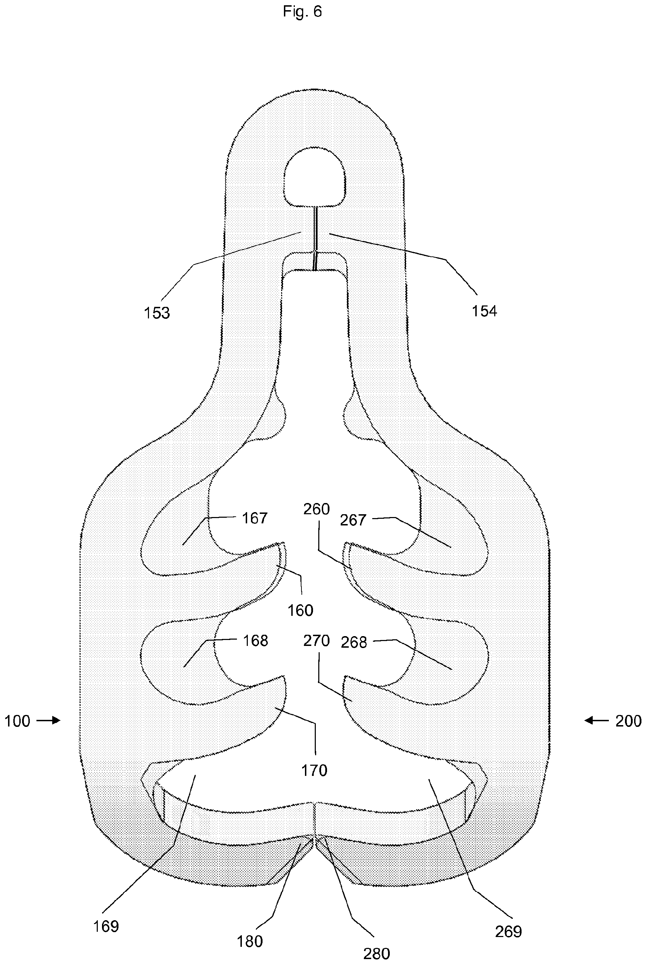

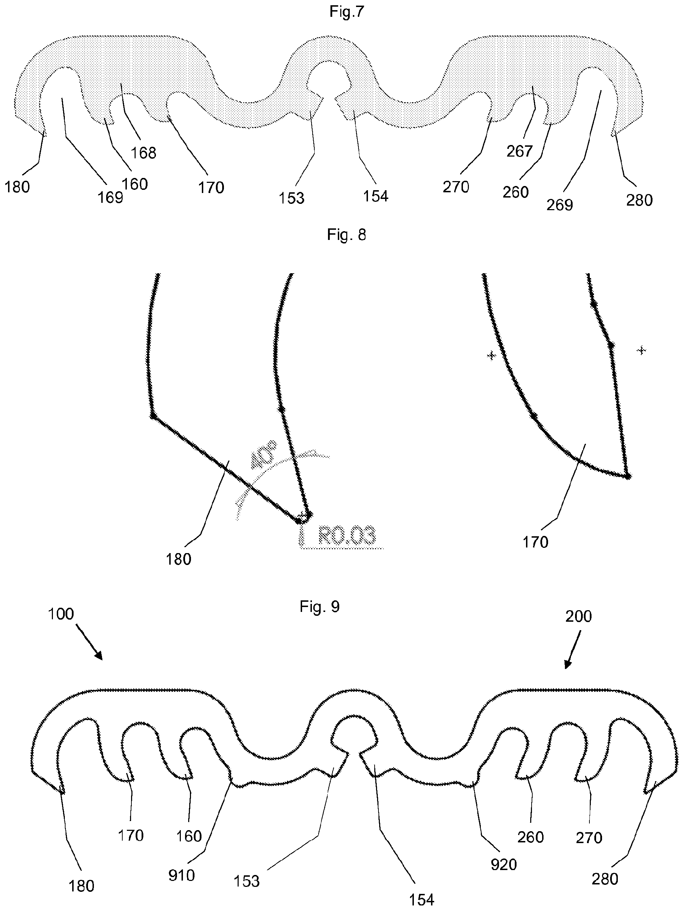

Les figures 6 et 7 représentent des vues de face respectivement en position ouverte et en position fermée. L'agrafe correspondant à cette variante est notamment destinée à l'hémostase. L'espace interne (168 ; 268) compris respectivement entre les dents de pincement (160, 170) et (260, 270) est partiellement "rempli" afin de réduire l'espace libre entre les bras (100, 200) de l'agrafe après fermeture et ainsi d'améliorer la compression des tissus sur lesquels le clip est positionné. L'espace (167, 267) jouxtant la dent (160, 260) la plus interne est également partiellement rempli.

L'espace (169, 269) jouxtant la dent (170, 270) la plus externe est quant lui laissé « creusé » afin d'assurer un bon ancrage de l'agrafe dans les tissus.

Le remplissage peut être partiel, avec un affinement progressif de l'épaisseur du matériau remplissant l'espace compris entre les deux dents consécutives.

La figure 8 représente une vue agrandie de l'extrémité pointue (180) de la branche (100) et la dent de pincement (170) la plus externe.

Dans une version préférée d'une agrafe dédiée à l'hémostase les extrémités des dents (180, 280) sont légèrement émoussées. Les dents externes doivent permettre d'ancrer l'agrafe au tissu en réduisant la pénétration. Avantageusement l'agrafe pourra ainsi être retirée par simple traction en produisant un déchirement superficiel minimal des tissus. Dans une variante préférée illustrée par la figure 8, les extrémités pointues (180, 280) présentent un angle d'attaque de 40° et un arrondi de 0.03 mm.

Dans la variante illustrée par la figure 9, chacun des bras (100, 200) présente un petit bossage de forme arrondie (910, 920). Son rôle est d'améliorer la compression et de rigidifier localement le bras. La torsion du métal à ce point sera réduite par le surcroit d'épaisseur et la déformation sera reportée au-delà du bossage. Le placement de ce bossage et l'ajustement de ses dimensions permettent de contrôler plus finement la géométrie du clip à la fermeture.

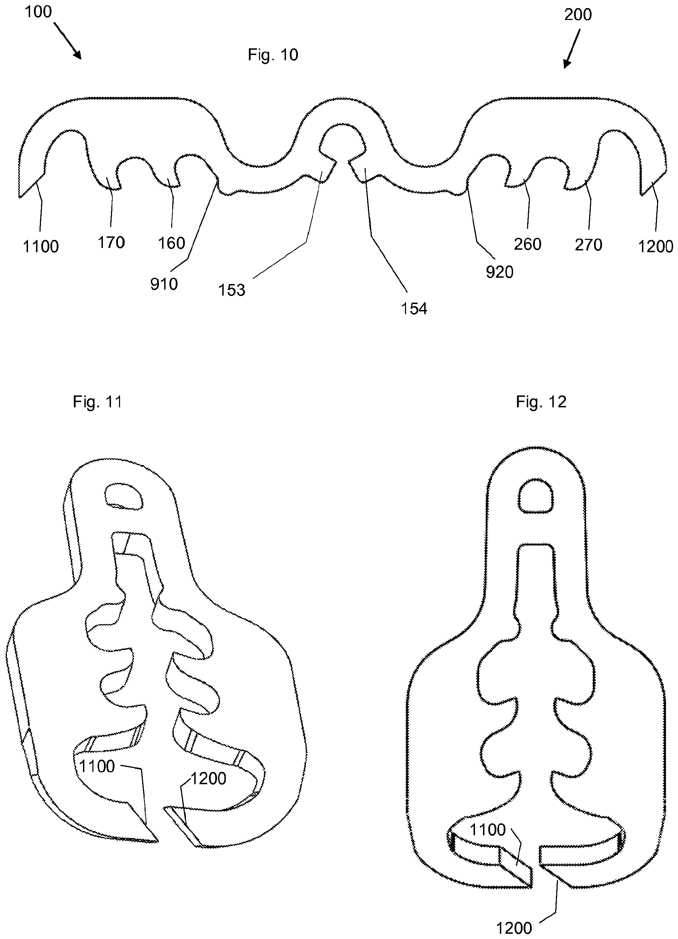

Dans la variante illustrée par les figures 10 à 12, les pointes d'accrochage externes (1100, 1200) ne sont pas symétriques. Le flanc de l'une est orienté vers l'extérieur et l'autre vers l'intérieur.

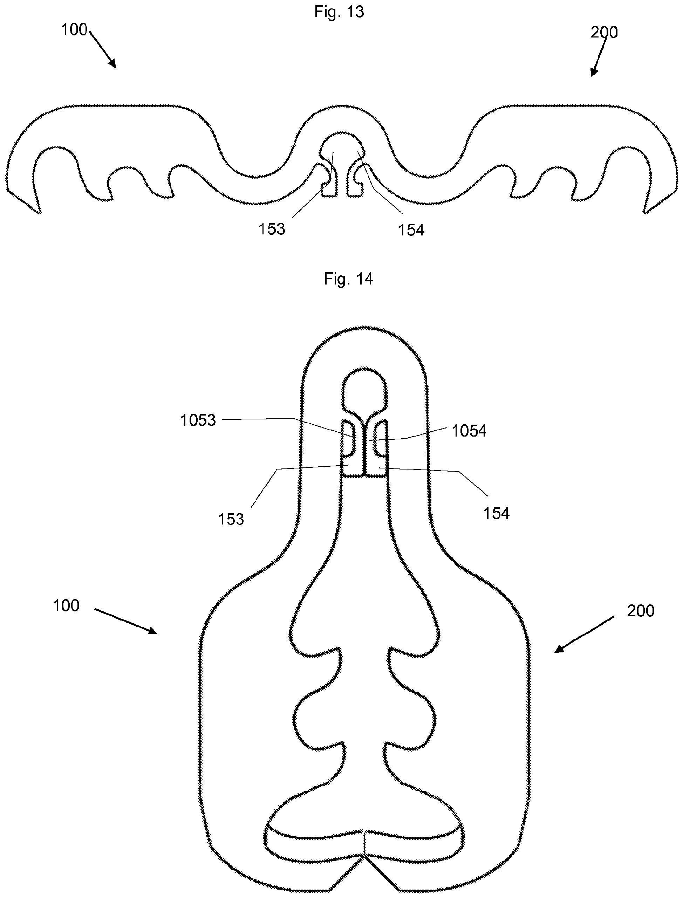

Dans la variante illustrée par les figures 13 et 14, les petits épaulements de blocage (153, 154) situés de part et d'autre de la zone d'accroche sont déportés au bout de petites pattes déformables (1053, 1054). Tout en conservant la fonction de maintien, l'espace dégagé par la courbure des pattes permet de limiter la friction avec le système de maintient au moment de la libération de l'épaulement.

### Enseignements issus des dessins

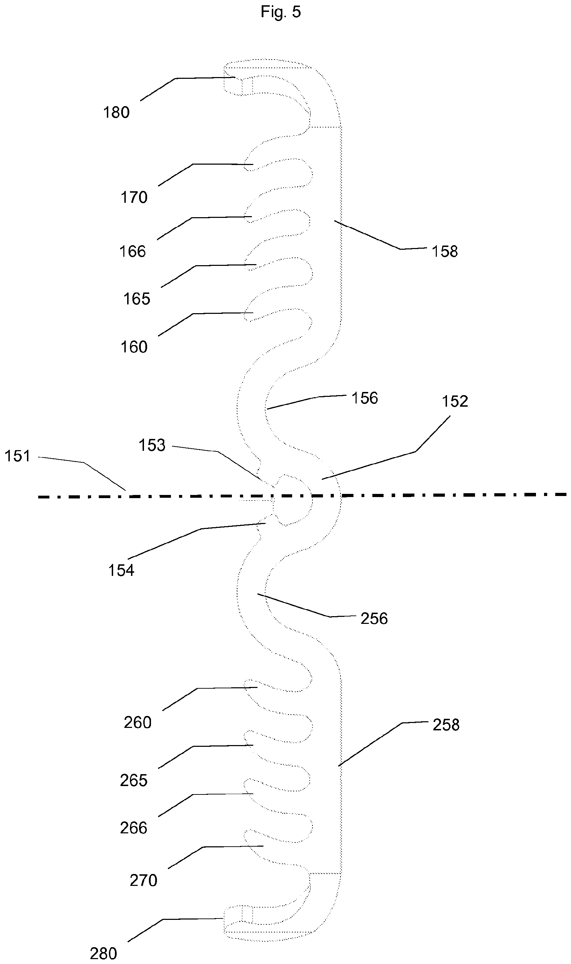

Il est précisé que les dessins correspondant aux figures 1 à 14 correspondent à des vues détaillées de variantes de réalisation réelles, et pas simplement des vues schématiques visant seulement à illustrer de manière approximative et schématique le principe de l'objet de l'invention. Les caractéristiques dimensionnelles et notamment les rapports dimensionnels, ainsi que les rayons de courbure, les formes des dents et des différentes zones de l'agrafe et l'orientation relative des différentes zones de l'agrafe apparaissant sur les dessins annexés ne sont pas fortuits, mais constitue un enseignement explicite de certains aspects secondaires de l'invention. Les dessins détaillés visent à fournir à l'homme du métier, dans le contexte de la description pris dans son ensemble, des caractéristiques qui ne sont éventuellement pas formulées textuellement et qui contribuent techniquement à apporter une solution au problème technique posé.

## Revendications

1. - Agrafe chirurgicale définissant une zone médiane (152) de pliage prolongée par une paire de branches (100, 200), chaque branche (100, 200) ayant une extrémité pointue d'accrochage (180, 280) et une zone intermédiaire de pincement située entre la zone médiane de pliage (152) et l'extrémité pointue d'accrochage (180, 280), l'agrafe étant déformable afin de plier chaque branche relativement à la zone médiane de pliage en amenant chaque extrémité d'accrochage à s'approcher de l'extrémité d'accrochage de l'autre branche, ladite agrafe étant constituée par une découpe métallique, d'épaisseur constante, lesdites zones intermédiaires de pincement de chacune desdites deux branches étant découpées pour former chacune au moins deux dents de pincement (160, 170 ; 260, 270) dont les tranches forment au moins deux surfaces complémentaires de pincement, parallèles au plan médian (151) lorsque l'agrafe est repliée, la section desdites surfaces de pincement correspondant à la section de ladite découpe métallique **caractérisée en ce que** l'espace interne (168 ; 268) compris respectivement entre les dents de pincement de chacune des branches (160, 170; 260 270) ainsi que l'espace (167, 267) jouxtant la dent (160, 260) la plus interne sont partiellement remplis afin de réduire l'espace libre entre les branches (100, 200) de l'agrafe après fermeture.

2. - Agrafe chirurgicale selon la revendication 1 **caractérisée en ce que** lesdites dents (160, , 170 ; 260, 270) présentent une forme arquée.

3. - Agrafe chirurgicale selon la revendication 1 **caractérisée en ce que** lesdites dents (160, 170 ; 260, 270) présentent, du coté dirigé vers l'extrémité pointue, une découpe arquée convexe.

4. - Agrafe chirurgicale selon la revendication 1 **caractérisée en ce que** lesdites dents (160, 170 ; 260, 270) présentent, du coté dirigé vers ladite zone médiane, une découpe arquée concave.

5. - Agrafe chirurgicale selon la revendication 1 **caractérisée en ce qu'**elle est configurée pour être déformée par pliage selon un axe de pivotement parallèle au plan transversal.

6. - Agrafe chirurgicale selon la revendication 1 **caractérisée en ce qu'**elle est déformée par pliage selon un axe de pivotement longitudinal et **en ce que** lesdites dents (160, 170 ; 260, 270) sont repliées dans un plan perpendiculaire au plan longitudinal.

7. - Agrafe chirurgicale selon la revendication 1 **caractérisée en ce que** la zone médiane de pliage présente une forme semi-circulaire avec des butées protubérantes (153, 154) s'étendant perpendiculairement à chacun des bras (100, 200), pour limiter l'angle de rapprochement des deux bras lors du repliement.

8. - Agrafe chirurgicale selon la revendication 1 **caractérisée en ce qu'**elle est configurée pour être déformée par pliage selon un axe de pivotement longitudinal et **en ce que** la zone médiane de pliage (152) présente une forme semi-circulaire avec des butées protubérantes s'étendant perpendiculairement à chacun des bras (100, 200), pour limiter l'angle de rapprochement des deux bras lors du repliement, lesdites butées étant repliées dans un plan perpendiculaire au plan longitudinal.

9. - Système constitué par une agrafe chirurgicale conforme à la revendication 1 et un applicateur adapté pour être inséré au travers du canal opérateur d'un endoscope flexible, et comportant un crochet (330) apte à coopérer avec la zone de pliage médiane (152), la zone médiane de pliage de l'agrafe étant un premier segment arqué, l'applicateur étant pourvu à son extrémité libre d'un manchon (300) présentant une surface annulaire (301) apte à former une butée contre laquelle viennent s'appuyer des épaulements (310, 320) formés par les surfaces de deuxièmes segments arqués (156, 256) de l'agrafe, pour provoquer le rapprochement des deux bras (100, 200) par un pivotement autour du centre du premier segment arqué (152), symétriquement par rapport au plan médian (151) lorsqu'on exerce une traction en direction opposée au manchon (300).

10. - Système selon la revendication précédente **caractérisé en ce que** l'intérieur d'un tube est crénelé afin de permettre la transmission de la rotation à l'agrafe en faisant entrer la gaine externe en rotation.

## Patentansprüche

1. Chirurgische Klammer, einen mittigen Biegebereich (152) definierend, von dem ein Paar Arme (100, 200) ausgeht, wobei jeder Arm (100, 200) ein spitzes Hakenende (180, 280) und einen zwischen dem mittigen Biegebereich (152) und dem spitzen Hakenende (180, 280) liegenden Klemmzwischenbereich aufweist, wobei die Klammer verformbar ist, so dass jeder Arm durch Annähern eines jeden Hakenendes an das Hakenende des anderen Arms bezüglich des mittigen Biegebereichs gebogen werden kann, wobei die Klammer aus einem Metallschneidstück konstanter Dicke besteht, wobei der Klemmzwischenbereich eines jeden der beiden Arme so geschnitten wurde, dass jeweils mindestens zwei Klemmzähne (160, 170; 260, 270) ausbildet sind, deren Ränder beim Zusammenbiegen der Klammer mindestens zwei zur Mittelebene (151) parallele komplementäre Klemmflächen bilden, wobei der Querschnitt der Klemmflächen dem Querschnitt des Metallschneidstücks entspricht, **dadurch gekennzeichnet, dass** der sich jeweils zwischen den Klemmzähnen eines jeden der Arme (160, 170; 260, 270) befindliche Innenraum (168; 268) sowie der an den innersten Zahn (160, 260) angrenzende Raum (167, 267) teilweise ausgefüllt sind, um den leeren Raum zwischen den Armen (100, 200) der Klammer nach dem Schließen zu verringern.

2. Chirurgische Klammer nach Anspruch 1, **dadurch gekennzeichnet, dass** die Zähne (160, 170; 260, 270) eine Bogenform aufweisen.

3. Chirurgische Klammer nach Anspruch 1, **dadurch gekennzeichnet, dass** die Zähne (160, 170; 260, 270) auf der dem spitzen Ende zugewandten Seite einen konvexen bogenförmigen Schnitt aufweisen.

4. Chirurgische Klammer nach Anspruch 1, **dadurch gekennzeichnet, dass** die Zähne (160, 170; 260, 270) auf der dem mittigen Bereich zugewandten Seite einen konkaven bogenförmigen Schnitt aufweisen.

5. Chirurgische Klammer nach Anspruch 1, **dadurch gekennzeichnet, dass** sie dazu eingerichtet ist, durch Biegen entlang einer zur Querebene parallelen Drehachse verformt zu werden.

6. Chirurgische Klammer nach Anspruch 1, **dadurch gekennzeichnet, dass** sie durch Biegen entlang einer längsverlaufenden Drehachse verformt wird und dass die Zähne (160, 170; 260, 270) in einer zur Längsebene senkrechten Ebene zusammengebogen werden.

7. Chirurgische Klammer nach Anspruch 1, **dadurch gekennzeichnet, dass** der mittige Biegebereich eine Halbkreisform mit hervorstehenden Anschlägen (153, 154) aufweist, die senkrecht von jedem der Arme (100, 200) abstehen, so dass der Annäherungswinkel der beiden Arme beim Zusammenbiegen begrenzt wird.

8. Chirurgische Klammer nach Anspruch 1, **dadurch gekennzeichnet, dass** sie dazu eingerichtet ist, durch Biegen entlang einer längsverlaufenden Drehachse verformt zu werden, und dass der mittige Biegebereich (152) eine Halbkreisform mit hervorstehenden Anschlägen aufweist, die senkrecht von jedem der Arme (100, 200) abstehen, so dass der Annäherungswinkel der beiden Arme beim Zusammenbiegen begrenzt wird, wobei die Anschläge in einer zur Längsebene senkrechten Ebene zusammengebogen werden.

9. System, bestehend aus einer chirurgischen Klammer nach Anspruch 1 und einem Applikator, der zum Einführen durch den Operationskanal eines biegsamen Endoskops ausgelegt ist und einen Haken (330) umfasst, der zum Zusammenwirken mit dem mittigen Biegebereich (152) geeignet ist, wobei der mittige Biegebereich der Klammer ein erstes bogenförmiges Segment ist, wobei der Applikator an seinem freien Ende mit einer Hülse (300) versehen ist, die eine ringförmigen Fläche (301) aufweist, die geeignet ist, einen Anschlag zu bilden, gegen den die von den Oberflächen der zweiten bogenförmigen Segmente (156, 256) der Klammer gebildeten Schultern (310, 320) zur Anlage kommen, so dass durch Drehen um die Mitte des ersten bogenförmigen Segments (152) eine bezüglich der Mittelebene (151) symmetrische Annäherung der beiden Arme (100, 200) bewirkt wird, wenn in zur Hülse (300) entgegengesetzter Richtung eine Zugkraft ausgeübt wird.

10. System nach dem vorhergehenden Anspruch, **dadurch gekennzeichnet, dass** das Innere eines Rohres gerieft ist, so dass durch das Versetzen der Außenhülle in Drehung die Übertragung der Drehung auf die Klammer ermöglicht wird.

## Claims

1. - Surgical staple defining a central folding region (152) extended by a pair of branches (100, 200), each branch (100, 200) having a pointed hooking end (180, 280) and an intermediate clamping region located between the central folding region (152) and the pointed hooking end (180, 280), the staple being deformable so that each branch can be folded relative to the central folding region by bringing each hooking end to approach the hooking end of the other branch, said staple being formed by a metal cutout which has a constant thickness, said intermediate clamping regions of each of said two branches being cut so as to each form at least two clamping teeth (160, 170; 260, 270) of which the edges form at least two complementary clamping surfaces parallel to the central plane (151) when the staple is folded, the cross section of said clamping surfaces corresponding to the cross section of said metal cutout, **characterized in that** the internal space (168; 268) in each case between the clamping teeth of each of the branches (160, 170; 260, 270) as well as the space (167, 267) adjoining the innermost tooth (160, 260) are partly filled in order to reduce the free space between the branches (100, 200) of the staple after closure.

2. - Surgical staple according to claim 1, **characterized in that** said teeth (160, 170; 260, 270) have an arcuate shape.

3. - Surgical staple according to claim 1, **characterized in that** said teeth (160, 170; 260, 270) have, on the side directed toward the pointed end, a convex arcuate cutout.

4. - Surgical staple according to claim 1, **characterized in that** said teeth (160, 170; 260, 270) have, on the side directed toward said central region, a concave arcuate cutout.

5. - Surgical staple according to claim 1, **characterized in that** it is configured to be deformed by being folded along a pivot axis parallel to the transverse plane.

6. - Surgical staple according to claim 1, **characterized in that** it is deformed by being folded along a longitudinal pivot axis, and **in that** said teeth (160, 170; 260, 270) are folded in a plane perpendicular to the longitudinal plane.

7. - Surgical staple according to claim 1, **characterized in that** the central folding region has a semi-circular shape having protruding stops (153, 154) which extend perpendicularly to each of the arms (100, 200) in order to limit the angle of approach of the two arms during folding.

8. - Surgical staple according to claim 1, **characterized in that** it is configured to be deformed by being folded along a longitudinal pivot axis, and **in that** the central folding region (152) has a semi-circular shape having protruding stops which extend perpendicularly to each of the arms (100, 200) in order to limit the angle of approach of the two arms during folding, said stops being folded in a plane perpendicular to the longitudinal plane.

9. - System, consisting of a surgical staple according to claim 1 and an applicator suitable for being inserted through the operating channel of a flexible endoscope, and comprising a hook (330) which is capable of interacting with the central folding region (152), the central folding region of the staple being a first arcuate segment, the applicator being provided, at its free end, with a sleeve (300) having an annular surface (301) which is capable of forming a stop against which the shoulders (310, 320), formed by the surfaces of second arcuate segments (156, 256) of the staple, come to rest in order to cause the approach of the two arms (100, 200) by pivoting about the center of the first arcuate segment (152) symmetrically with respect to the central plane (151) when traction is exerted in the opposite direction to the sleeve (300).

10. - System according to the preceding claim, **characterized in that** the inside of a tube is crenellated in order to allow the transmission of the rotation to the staple by making the outer sheath rotate.
